(19) Europäisches Patentamt
European Patent Office
Office européen des brevets

(11) EP 1 559 420 A1

(12) EUROPEAN PATENT APPLICATION

(43) Date of publication:
03.08.2005 Bulletin 2005/31

(51) Int Cl.⁷: **A61K 31/00**, A61K 31/475, A61K 38/10, A61P 27/16

(21) Application number: **05009885.4**

(22) Date of filing: **21.09.2001**

(84) Designated Contracting States:
**AT BE CH CY DE DK ES FI FR GB GR IE IT LI LU MC NL PT SE TR**

(30) Priority: **21.09.2000 EP 00120652**

(62) Document number(s) of the earlier application(s) in accordance with Art. 76 EPC:
**01978382.8 / 1 331 931**

(71) Applicant: **Tinnitus Forschungs- und Entwicklungs GmbH**
**80639 München (DE)**

(72) Inventor: **The designation of the inventor has not yet been filed**

(74) Representative:
**Mütschele, Thomas, Dr. Dipl.-Chem.**
**Patentanwälte Ruff, Wilhelm**
**Beier, Dauster & Partner**
**Kronenstrasse 30**
**70174 Stuttgart (DE)**

Remarks:
This application was filed on 06 - 05 - 2005 as a divisional application to the application mentioned under INID code 62.

# (54) Treatment of tinnitus

(57) The invention relates to the use of substances which are at least partly blocking one ionotropic acetylcholine receptor of the inner ear and/or a calcium-activated potassium channel functionally associated with said acetylcholine receptor of the inner ear for the manufacturing of a pharmaceutical composition or medicament for the treatment of tinnitus.

EP 1 559 420 A1

## Description

The invention relates to the use of substances for the manufacturing of a pharmaceutical composition or medicament for the treatment of tinnitus.

Two main cell types are involved in the processing of sound. The inner hair cells which work as transducers to the central nervous system (CNS) and the outer hair cells which are electro-mechanical amplifiers for sound on the basis of a loudspeaker-like-mechanism. The outer hair cells (OHC) receive synaptic input from the superior olive that controls cochlear afferent activity by inhibiting the fast voltage-dependent amplification mechanism provided by these sensory cells (Guinan, J. 1996. Dallos, P., Popper, N. and Fay, R. eds. New York: Springer, 435-502). This fast synaptic inhibition is generally mediated by a hyperpolarizing chlorid conductance through different receptors activated by transmitters released from the presynapse (Alger, B.E. 1991. Ann. NY Acad. Sci. 627, 249-263; Betz, H., Kuhse, J., Schmieden, V., Laube, B., Kirsch, J. and Harvey, R.J. 1999. Ann. NY Acad. Sci. 868, 667-676). How this synaptic inhibition in OHCs works is still unclear. What is known is that neuronal acetylcholine receptors (nAChR) in OHCs supply the prosynaptic cell with calcium ($Ca^{2+}$) that initiates an inhibitory hyperpolarization by opening a calcium$^{2+}$-activated potassium (SK) channel (Oliver, D., Klöcker, N., Schluck, J., Baukrowitz, T., Ruppersberg, J.P. and Fakler, B. 2000. Neuron 26, 595-601).

Damages of the inner ear occur e.g. by traumatic, hypoxic and toxic influences. The main reasons for that damages are noise, sudden hearing loss and drugs e.g. antibiotics or cytostatics. Diseases or disturbances in which subjective noise in the ear, a so-called tinnitus occurs, are widespread. It is estimated that in Germany alone roughly six million people suffer from tinnitus. In roughly 800.000 cases the tinnitus is so pronounced that these patients need intensive treatment by a physician, due to the patient being seriously handicapped by tormenting ear noise.

In the case of tinnitus different medical therapies have been proposed hitherto. These include, in addition to the use of anaesthetica, the application of lidocaine-type antiarrhythmica or anticonvulsiva, or infusion therapy. However, in most cases treatments of this kind fail to bring satisfactory results.

In the European Patent 0759295 it was shown, that adamantane derivatives can be successfully used for the treatment of tinnitus which is associated with a so-called positive recruitment and/or a reduction or a failure of otoacustic emissions. However, the molecular mechanism how adamantane derivative works was still unknown, although there was evidence that receptors present on the outer hair cells may be involved. In the publication of Oliver et al. (Oliver, D. et al., 2000, see above) it was shown that the hyperpolarization of OHC cells is mediated by SK2 channels and that it occurs rapidly, explaining a possible mechanism for the generation of fast inhibitory synaptic transmission.

The object of the invention is to find new targets and to make available new substance classes for the treatment of tinnitus by definitely explaining the molecular mechanism involved in the signaltransmission of the OHCs which might be a reason for the pathomechanism of tinnitus. This object is solved by the subject-matter of the independent claims 1 and 10. Preferred embodiments are given in the dependent claims 2 to 9 and 11 to 17. The wording of all these claims is hereby incorporated into the content of the description by reference.

Surprisingly it was found that neuronal acetylcholine receptors of the inner ear comprise alpha9- and alpha10-subunits. Therefore, there is a alpha9/alpha10 heteromeric acetylcholine receptor. Via such acetylcholine receptors the hyperpolarization of outer hair cells (OHCs) occurs and it is mediated by SK2-channels. The acetylcholine receptor can be blocked efficiently by memantine (3.5-dimethyl-1-adamantan-amine) and other substances and therefore an inactivation of the OHCs occurs. Furthermore, it was found that this ionotropic acetylcholine receptor is functionally associated with a calcium-activated potassium channel. Blocking of this channel can also lead to the inactivation of the OHCs. These new findings make it possible to use the acetylcholine receptor and/or the SK-channel as a new target for the treatment of tinnitus. Accordingly, new substance classes can be used blocking the acetylcholine receptor and/or the calcium-activated potassium channel.

The potential pathomechanism of tinnitus may also be explained by these findings. The OHC may physiologically or pathologically generate the psychical impression of sound by undergoing mechanical movements which are transmitted to the inner hair cells and through those propagated to the CNS. Such movements are caused by membrane potential changes caused either by sound or by the efferent synapse of the OHC. This synapse causes membrane potential changes which are not associated with real sound but interpreted by the CNS as pathological sound (tinnitus). As nAChR and the calcium-activated potassium channel are involved in this signal transmission of this synapse, any potent inhibitor of the signal transmission might inhibit tinnitus.

According to the invention, at least one substance is used for the manufacturing of a pharmaceutical composition or medicament for the treatment of tinnitus, wherein said substance is at least partly blocking at least one ionotropic acetylcholine receptor of the inner ear and wherein that substance is not an adamantane derivative according to the following formula

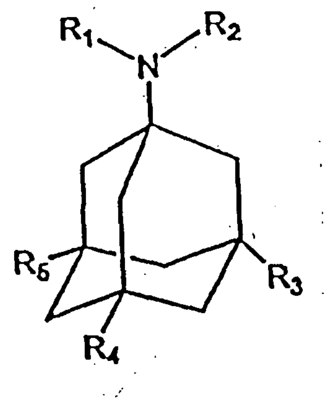

where $R_1$ and $R_2$

- are the same or different, and include hydrogen or straight or branched chain alkyl groups having 1 to 6 C atoms, or
- together with the N atom present a heterocyclic group having 5 or 6 ring atoms,

where $R_3$ and $R_4$ are the same or different, and include hydrogen, straight, or branched chain alkyl groups having 1 to 6 C atoms, cycloalkyl groups having 5 to 6 C atoms, or phenyl, and

where $R_5$ is hydrogen or a straight or branched chain alkyl group having 1 to 6 C atoms.

The substance can optionally be used in the form of its pharmaceutically acceptable salts and/or optionally together with a pharmaceutically carrier.

According to the invention, this ionotropic acetylcholine receptor of the inner ear comprises at least one so-called alpha9-subunit. As an alternative or preferably in addition the receptor comprises at least one so-called alpha10-sub-unit. Preferably the receptor is functionally associated with at least one calcium-activated potassium channel. This calcium-activated potassium channel is preferably of SK (small conductance) subtype, wherein preferably that SK potassium channel is of the SK2 subtype.

According to the invention the substance used for the treatment of tinnitus can be a strychnine derivative, a peptide, preferably a polypeptide, or a polynucleotide encoding such peptide, preferably a polypeptide. Furthermore, the substance can be a small molecular compound, preferably a small molecular compound with a molecular weight (MW) < 1000.

According to further embodiments the invention comprises further the use of a substance for the manufacturing of a pharmaceutical composition or medicament for the treatment of tinnitus, wherein said substance is at least partly blocking at least one calcium-activated potassium channel functionally associated with an ionotropic acetylcholine receptor of the inner ear and wherein that substance is not an adamantane derivative according to the following formula

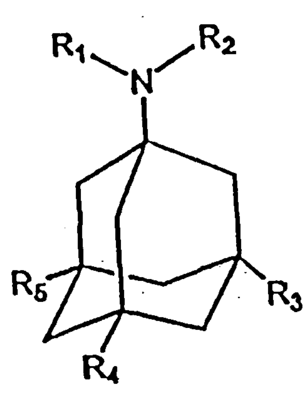

where $R_1$ and $R_2$

- are the same or different, and include hydrogen or straight or branched chain alkyl groups having 1 to 6 C atoms, or
- together with the N atom present a heterocyclic group having 5 or 6 ring atoms,

where $R_3$ and $R_4$ are the same or different, and include hydrogen, straight, or branched chain alkyl groups having 1 to 6 C atoms, cycloalkyl groups having 5 to 6 C atoms, or phenyl, and

where $R_5$ is hydrogen or a straight or branched chain alkyl group having 1 to 6 C atoms.

As described above, the substance can optionally be used in the form of its pharmaceutically acceptable salts and/or together with a pharmaceutically carrier.

The ionotropic acetylcholine receptor which is functionally associated with the calcium-activated potassium channel comprises according to these embodiments of the invention at least one so-called alpha9-subunit. As an alternative or preferably in addition this acetylcholine receptor comprises at least one so-called alpha10-subunit. The calcium-activated potassium channel is preferably of the SK subtype, wherein preferably said SK potassium channel is of the SK2 subtype.

Finally, according to the invention the substance which at least partly blocks at least one calcium-activated potassium channel defined as above can be a peptide, preferably a polypeptide. This polypeptide can be apamine, which preferably blocks this channel, but also even up to now unknown compounds which block this channel are claimed for the inventive use. The substance can also be a polynucleotide encoding such a peptide, preferably polypeptide, or it can be a small molecular compound, preferably a small molecular compound with a molecular weight (MW) < 1000.

The described features and further features of the invention can be gathered from the following description of preferred embodiments in conjunction with the subclaims. The individual features can be implemented separately or in the form of subcombinations.

## MATERIALS AND METHODS

### Patch-clamp recordings on outer hair cells

The apical turn of the organ of Corti was dissected from cochleae of three to six week old Wistar rats as described previously (Oliver et al., 1999, J Physiol (Lond) 519, 791-800; Oliver et al., 2000, Neuron 26, 595-601). The preparation was performed in a solution containing (in mM) 144 NaCl, 5.8 KCl, 0.1 $CaCl_2$, 2.1 $MgCl_2$, 10 HEPES, 0.7 $Na_2HPO_4$, 5.6 glucose, pH adjusted to 7.3 with NaOH. For recordings, OHCs located between half and one turn from the apex of the cochlea were chosen. If necessary, supporting cells were removed with gentle suction from a cleaning pipette carefully avoiding mechanical disturbance of the efferent nerve terminals.
Whole-cell patch-clamp recordings were performed with an Axopatch 200B amplifier (Axon Instruments, Foster City, CA) at room temperature (22 - 25°C). Electrodes were pulled from quartz glass, had resistances of 2-3 MΩ and were filled with intracellular solution (in mM): 135 KCl, 3.5 $MgCl_2$, 0.1 $CaCl_2$, 5 EGTA, 5 HEPES, 2.5 $Na_2ATP$. The pH of the solution was adjusted to pH 7.3 with KOH. Membrane potential was corrected for the electrode junction potential (-4 mV). Whole-cell series resistance ranged from 4 to 9 MΩ and was not compensated. Currents were filtered at 1 kHz and sampled at 5 kHz.
The specimen were continuously superfused with extracellular solution (in mM: 144 NaCl, 5.8 KCl, 2 $CaCl_2$, 0.9 $MgCl_2$, 10 HEPES, 0.7 $Na_2HPO_4$, 5.6 glucose, pH adjusted to 7.3 with NaOH). Chemicals as well as depolarizing solutions were applied via a glass capillary (diameter approximately 80 μm) positioned close to the organ of Corti. For the depolarizing external solution KCl was substituted for an equal amount of NaCl to result in $[K^+]_{ex}$ of 47 mM. Strychnine, apamine and acetylcholine were added to the extracellular solution from aqueous stock solutions. To block the large OHC resting $K^+$ current, $I_{k,n}$, 10 μM linopirdine (RBI) or 1 - 5 μM XE991 (obtained from DuPont) was added to the standard extracellular medium from stock solutions made with DMSO (final concentration < 0.1 %) (Housley and Ashmore, 1992, J Physiol (Lond) 448, 73-98; Marcotti and Kros, 1999, J Physiol (Lond) 520, 653-660). XE991 is a M-current blocker (Wang et al., 1998, Science 282, 1890-1893) that inhibits $I_{k,n}$ at submicromolar concentrations in a poorly reversible manner.

### Electrophysiology on heterologously expressed channels

In vitro mRNA synthesis and oocyte injections were performed as previously described (Fakler et al., 1995, Cell 80, 149-154; Oliver et al., 2000, see above). The electrophysiology on oocytes was also as described previously (Oliver et al., 2000, see above). In detail, AChR subunits and SK2 channels were heterologously expressed in *Xenopus* oocytes. Oocytes were surgically removed from adult females and dissected manually. 4 - 5 day prior to electrophysiological recordings, Dumont stage VI oocytes were injected with about 50 ng RNA. For coexpression experiments, the total amount of RNA was kept constant and the different RNAs were coinjected in equal concentrations. Two-electrode voltage-clamp measurements were performed with a TurboTec 01C amplifier (npi, Tamm, Germany), using microelectrodes of 0.1 to 0.5 MΩ filled with 3 M KCl. Extracellular medium was CaNFR, containing (in mM): 120 KCl, 2.5 KCl, 2 $CaCl_2$, 10 HEPES, pH adjusted to 7.3 with NaOH. For experiments in the absence of extracellular $Ca^{2+}$, the

extracellular solution was MgNFR (in mM): 120 KCl, 2.5 KCl, 2 $MgCl_2$, 10 HEPES, pH adjusted to 7.3 with NaOH. ACh was added from stock solutions and applied through an application system, that allowed solution exchange with a time constant of ~1s. Currents were filtered at 100 Hz and sampled at a frequency of 1 kHz. Dose-inhibition relations obtained from electrophysiological experiments were fitted to the empirical Hill equation,

$$I_{norm} = \frac{1}{1+\left(\frac{c}{IC_{50}}\right)^{n_H}} \quad (1)$$

where $I_{norm}$ is the normalized current, c is the blocker concentration, $IC_{50}$ is the half-inhibitory concentration, and $n_H$ is the Hill coefficient.

Data analysis and fitting was performed with IgorPro (Wavemetrics, Lake Oswego, OR) on a Macintosh PowerPC. Unless stated otherwise, data are presented as mean ± standard deviation.

**Molecular biology**

The coding region of the rat α10 gene (GenBank Accession No. AF196344) was amplified from rat brain cDNA by PCR using 5'- and 3'-adapter-primers containing suitable restriction sites (GAGACCCGGGAGCTCCACC, ATGGGGACAAGGAGCCACTACC and GAGTCTAGATTACAGGGCTTGCACCAGTACAATG). The amplified fragments were subcloned into the *Xenopus* oocyte expression vector pGEM-HE (gift of Dr. J. Tytgat), yielding pGEM-HE-nAChR-α10, verified by sequencing. Capped mRNAs for α9, α10 and SK2 were synthesized *in vitro* using the mMESSAGE mMACHINE kit (Ambion, Austin, TX).

To detect α10 and α9 transcripts, PCR was performed using reverse transcribed RNA isolated from either OHCs (containing some Deiters cells) or supporting cells (Deiters and Hensen's cells) as template. Cells were collected from rat organs of Corti using suction glass micropipettes (diameter 10 µm). RNA was prepared from ~100 cells of each fraction using the Qiagen RNeasy kit (Qiagen, Hilden, FRG) according to the manufacturers instructions. The oligonucleotides used as primers in the PCR reactions were chosen to span an intron in the human α9 and α10 genes to allow differentiation between products originating from cDNA and products originating from contaminating genomic DNA.

α9 sense: CGTCCTCATATCGTTCCTCGCTCCG,
α9 antisense: TGGTAAGGGCTGTGGAGGCAGTGA;

α10 sense: GCAGCCTACGTGTGCAACCTCCTGC,
α10 antisense: AGGTGTCCCAGCAGGAGAACCCGAG.

For each PCR reaction, RNA corresponding to -3 cells was used as template.

Figure legends:

Fig. 1: (A) Model for the inhibitory synaptic transmission at outer hair cells (OHCs) as derived from experiments with isolated cells. (B) Inhibitory postsynaptic currents (IPSCs) were inhibited by strychnine and apamine.

Fig. 2: (A) ACh (100 µM) induced inward currents in oocytes coding for α9 but not in oocytes coding for α10. (B) Detection of α9 and α10 mRNA in OHCs by RT-PCR. (C) Same experiment as in (A), but for coexpression of both subunits. (D) Current amplitudes from experiments as in (A) and (C) summarized for oocytes injected with RNA coding for a non-conducting SK2 channel mutant (control), α9, α10, and α9/α10.

Fig. 3: (A) Currents evoked by activation of α9/α10 nAChRs are dependent on external $Ca^{2+}$. (B) Currents from homomeric α9 and heteromeric α9/α10 nAChRs recorded by application of 100 μM ACh in nominally $Ca^{2+}$-free external solution. (C) Current amplitudes from oocytes expressing α9 and α9/α10, recorded as in figure 3B.

Fig. 4: Application of 100 μM ACh for the time indicated to an oocyte coexpressing α9, α10, and SK2.

Fig. 5: Block of alpha9/alpha10 SK2 current responses to acetylcholine in Xenopus oocytes by strychnine.

**Experiment 1:**

In Fig. 1A, a model is shown of how the inhibitory transmission at OHCs occurs. $Ca^{2+}$ entering the postsynapse via the transmitter-activated nAChR opens SK channels and thus results in hyperpolarizing $K^+$ currents (Art, J.J., Fettiplace, R. and Fuchs, P.A. 1984. J. Physiol. (Lond.) 356, 525-550; Yuhas, W.A. and Fuchs, P.A. 1999. J. Comp. Physiol. 18, 455-462).

In Fig 1B, postsynaptic currents were recorded from OHCs in voltage-clamp experiments when cells were held at -64 mV and the whole Corti preparation was superfused with high extracellular $K^+$ (150 mM) to depolarize the presynapse. Application of high $K^+$ and toxins as indicated by horizontal bars: current and time scaling as indicated.

Consistent with the model presented in Fig. 1A, the postsynaptic currents were inhibited by application of either the acetylcholine blocker strychnine (Elgoyhen, A.B., Johnson, D.S., Boutler, J., Vetter, D.E. and Heinemann, S. 1994. Cell 79, 705-715) or the SK channel blocker apamine (complete inhibition at 100 nM and 10 nM, respectively; Fig. 1B).

**Experiment 2:**

α9 and α10 nAChR subunits are expressed in OHCs and coassemble to functional channels. In (A) ACh (100 μM) induced inward currents in oocytes injected with RNA coding for α9 (lower trace) but not in oocytes injected with RNA coding for α10 (upper trace). Holding potential was - 80 mV. Fast downward spikes in the traces are artifacts resulting from switching between solutions. (B) Detection of α10 and α9 mRNA in OHCs by RT-PCR. Fragments of the expected length were amplified for α9 and α10 from OHCs (lanes 1, 2) but not from supporting cells (lane 3, data for α9 not shown). Controls were OHC-RNA without RT added (lane 4) and water (lane 5) as a template for PCR. (C) Same experiment as in (A), but for coexpression of both subunits. Note the different current scaling. The recording from (A) (α9) was added for comparison. (D) Current amplitudes from experiments as in (A) and (C) summarized for oocytes injected with RNA coding for a non-conducting SK2 channel mutant (control), α9, α10, and α9/α10 (values are mean ± standard error of 5, 13, and 18 oocytes, respectively).

The nAChR of outer hair cells has been shown to contain the α9 subunit by a variety of methods including *in-situ* hybridization (Elgoyhen et al., 1994, see above; Morley et al., 1998, Brain Res Mol Brain Res 53, 78-87) single-cell RT-PCR (Glowatzki and Fuchs., 1995, Science 288, 2366-2368), transgenic expression of green fluorescent protein controlled by the α9 promotor (Zuo et al., 1999, Proc Natl Acad Sci 96, 14100-14105), and inactivation of the α9 gene (Vetter et al., 1999, Neuron 23, 93-103). Homomeric α9 receptors yield remarkably small currents when heterologously expressed in Xenopus oocytes (Elgoyhen et al., 1994, see above), raising the possibility that an additional subunit is needed to yield the fully functional OHC receptor. However, OHCs lack any other of the known nAChR subtypes (Morley et al., 1998, see above). A GenBank search yielded a new subunit of the nAChR family (GenBank Accession No. AF196344), designated as α10.

Therefore, to test whether the inhibition of the complex IPSCs resulted from block of $Ca^{2+}$ -entry via the nAChR and to test if α10 is a candidate subunit for the OHC receptor, OHC nAChR expressed heterologously in *Xenopus* oocytes were tested. However, applications of 100 μM ACh to oocytes injected with α9-specific RNA yielded very small currents (9.3 + 5.0 nA at -80 mV; Fig. 2A), consistent with previous reports (Elgoyhen et al., 1994, see above; Katz et al., 2000, Hear Res 141, 117-128). No currents exceeding background levels were observed with the rat homologue of the α10 subunit, a member of the nAChR family recently identified by Boulter and colleagues (GenBank Accession No. AF196344; Fig. 2A, D). As shown in Fig. 2B by RT-PCR on OHCs isolated from the rat organ of Corti (see Methods), α10 mRNA is indeed present in these sensory cells, while it was not detected in the supporting cell fraction, containing Hensen and Deiters cells. In a control experiment with RNA from OHCs that was not reverse transcribed, PCR amplified a fragment of -900 bp (Fig. 2B, lane 4) which most likely resulted from contamination with genomic DNA as the length of this fragment is in good agreement with the sequence defined by the primer pair in the human genome (BAC from chromosome 11; GenBank Acc.#AC060812). When both, α9 and α10, were coexpressed in oocytes, large inward currents with peak amplitudes of up to -35 μA (at -80 mV) were recorded upon application of ACh (Fig. 2C, D). Similar to α9-mediated currents, the timecourse was characterized by an initial transient declining to a smaller plateau of a variable aplitude with respect to the peak current.

The increase in current amplitude of more than 3 orders of magnitude (compared to homomeric α9 expression) together with the coexpression of α9 and α10 in OHCs suggest that heteromultimerization of both subunits is essential to give fully functional receptor channels. Moreover, the absence of any other of the known nAChR subunits (Morley et al., 1998, see above) strongly suggests that the OHC nAChR is a heteromer composed of α9 and α10 subunits.

**Experiment 3:**

In (A) currents evoked by activation of α9/α10 nAChRs are dependent on external $Ca^{2+}$. Traces show subsequent applications of 100 μM ACh to the same oocyte in CaNFR ($Ca^{2+}$) and MgNFR ($Mg^{2+}$) at -80 mV. (B) Currents from homomeric α9 (upper trace) and heteromeric α9/α10 nAChRs (lower trace), recorded by application of 100 μM ACh in nominally $Ca^{2+}$-free external solution (MgNFR; -80 mV). (C) Current amplitudes from oocytes expressing α9 and α9/α10, recorded as in figure 3B (mean ± standard error from 12 and 15 oocytes, respectively).

A characteristic feature of homomeric α9 channels is their exceptionally high $Ca^{2+}$-permeability (Jagger et al., 2000, J Physiol 527, 49-54; Katz et al., see above). This $Ca^{2+}$-permeability is thought to be essential for the OHC nAChR, since it allows for a $Ca^{2+}$ influx sufficiently high to effectively activate SK-type potassium channels. The oocyte expression system, however, is characterized by high endogenous expression levels of $Ca^{2+}$-activated $Cl^-$channels (Stühmer and Parekh, 1995, in Single-channel recording 2nd edition (Neher E and Sakmann B eds) 341-356, Plenum Press, New York). Therefore, opening of $Ca^{2+}$-permeable channels in an external medium containing $Ca^{2+}$ leads to a coactivation of a $Cl^-$ conductance. When external $Ca^{2+}$ was substituted for $Mg^{2+}$, currents induced by ACh application onto α9/α10 heteromeric channels were reduced by a factor of roughly 10 (Fig. 3A). Thus, a large fraction of the ACh-induced current measured in CaNFR was due to opening of $Ca^{2+}$-activated $Cl^-$-currents. This was also supported by the reversal potential of the current in CaNFR of about - 25 mV (data not shown), close to the estimated $Cl^-$ equilibrium potential in *Xenopus* oocytes (Stühmer and Parekh, 1995, see above). Consequently, α9/α10 heteromeric channels had a significant $Ca^{2+}$-permeability, similar to what is known from homomeric α9 receptors. Application of ACh in the absence of extracellular $Ca^{2+}$ allowed the recording of α9/α10 currents in isolation. Heteromeric channels yielded currents that were 100fold larger than currents recorded from α9 channels under the same conditions, confirming the large gain of receptor conductance by coexpression that was observed in the presence of external $Ca^{2+}$ (Fig. 3B, C). In the absence of $Ca^{2+}$, α9/α10 also showed consistent kinetics characterized by slow desensitization on a time scale of seconds. Desensitization was not observed with α9 channels within the limits of the speed of solution exchange (Fig. 3B).

**Experiment 4:**

Application of 100 μM ACh for the time indicated to an oocyte coexpressing α9, α10, and SK2. Traces are subsequent recordings from the same oocyte at the holding potentials indicated.

In hair cells, $Ca^{2+}$ influx via nAChRs activates SK2 channels to give rise to IPSCs. Figure 4 shows, that this activation cascade may be reconstituted in *Xenopus* oocytes by coexpression of the α9/α10 nAChR with SK2 channels. In oocytes expressing both channel species, application of ACh evoked a biphasic response at -70 mV. An initial inward current carried mainly by chloride was followed by an outward current due to the activation of SK2 channels (Fig. 4). With the $Cl^-$ driving force largely abolished and an increased driving force for $K^+$ at a membrane potential of -30 mV, ACh induced a monophasic potassium outward current, similar to the response of isolated OHCs to ACh application (Blanchet et al., 1996, J Neurosci 16, 2574-2584; Evans, 1996, J Physiol (Lond) 491, 563-578).

**Experiment 5:**

For further characterization of strychnine treatment on the nAChR, the mRNA of alpha9/alpha10 subunits of the acetylcholine receptor were injected in oocytes of Xenopus laevis. After expression of these subunits, they were investigated by treatment with strychnine in voltage-clamp experiment. The cells were held at -30 mV. Current and time scaling are as indicated. As is shown in Fig. 5, similar results were obtained as in Fig. 1. Consistent with the model shown above (Fig. 1), the current responses at the acetylcholine receptor could be blocked reversibly by application with the acetylcholine blocker strychnine. A complete inhibition occurs at 1 μM, and after washout of strychnine for 3 min. the receptor was again functionally active, indicating a reversible block of the receptor.

## Claims

1. Use of a substance for the manufacturing of a pharmaceutical composition or medicament for the treatment of tinnitus, wherein said substance is at least partly blocking at least one ionotropic acetylcholine receptor of the inner

ear and wherein said substance is not an adamantane derivative according to the following formula

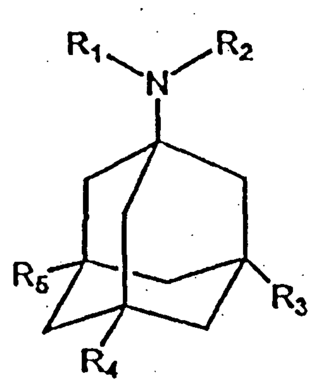

where $R_1$ and $R_2$

- are the same or different, and include hydrogen or straight or branched chain alkyl groups having 1 to 6 C atoms, or
- together with the N atom present a heterocyclic group having 5 or 6 ring atoms, where $R_3$ and $R_4$ are the same or different, and include hydrogen, straight or branched chain alkyl groups having 1 to 6 C atoms, cycloalkyl groups having 5 to 6 C atoms, or phenyl, and

where $R_5$ is hydrogen or a straight or branched chain alkyl group having 1 to 6 C atoms.

**2.** Use according to claim 1, **characterized in that** said acetylcholine receptor comprises at least one alpha9-subunit.

**3.** Use according to claim 1 or claim 2, **characterized in that** said acetylcholine receptor comprises at least one alpha10-subunit.

**4.** Use according to one of the preceding claims, **characterized in that** said acetylcholine receptor is functionally associated with at least one calcium-activated potassium channel.

**5.** Use according to claim 4, **characterized in that** said calcium-activated potassium channel is of SK subtype, wherein preferably said SK potassium channel is SK2.

**6.** Use according to one of the preceding claims, **characterized in that** said substance is a strychnine derivative.

**7.** Use according to one of claims 1 to 5, **characterized in that** said substance is a peptide, preferably a polypeptide.

**8.** Use according to one of claims 1 to 5, **characterized in that** said substance is a polynucleotide encoding a peptide, preferably a polypeptide, of claim 7.

**9.** Use according to one of the preceding claims, **characterized in that** said substance is a small molecular compound, preferably a small molecular compound with a molecular weight (MW) < 1000.

**10.** Use of a substance for the manufacturing of a pharmaceutical composition or medicament for the treatment of tinnitus, wherein said substance is at least partly blocking at least one calcium-activated potassium channel functionally associated with an ionotropic acetylcholine receptor of the inner ear and wherein said substance is not an adamantane derivative according to the following formula

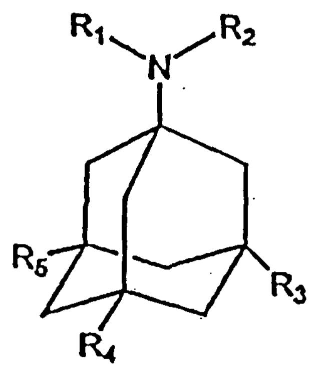

where $R_1$ and $R_2$

- are the same or different, and include hydrogen or straight or branched chain alkyl groups having 1 to 6 C atoms, or
- together with the N atom present a heterocyclic group having 5 or 6 ring atoms, where $R_3$ and $R_4$ are the same or different, and include hydrogen, straight or branched chain alkyl groups having 1 to 6 C atoms, cycloalkyl groups having 5 to 6 C atoms, or phenyl, and

where $R_5$ is hydrogen or a straight or branched chain alkyl group having 1 to 6 C atoms.

**11.** Use according to claim 10, **characterized in that** said acetylcholine receptor comprises at least one alpha9-subunit.

**12.** Use according to claim 10 or claim 11, **characterized in that** said acetylcholine receptor comprises at least one alpha10-subunit.

**13.** Use according to one of claims 10 to 12, **characterized in that** said calcium-activated potassium channel is of SK subtype, wherein preferably said SK potassium channel is SK2.

**14.** Use according to one of claims 10 to 13, **characterized in that** said substance is a peptide, preferably a polypeptide.

**15.** Use according to claim 14, **characterized in that** said peptide is apamine.

**16.** Use according to one of claims 10 to 13, **characterized in that** said substance is a polynucleotide encoding a peptide, preferably a polypeptide, of claim 14.

**17.** Use according to one of claims 10 to 16, **characterized in that** said substance is a small molecular compound, preferably a small molecular compound with a molecular weight (MW) < 1000.

Fig. 1

A

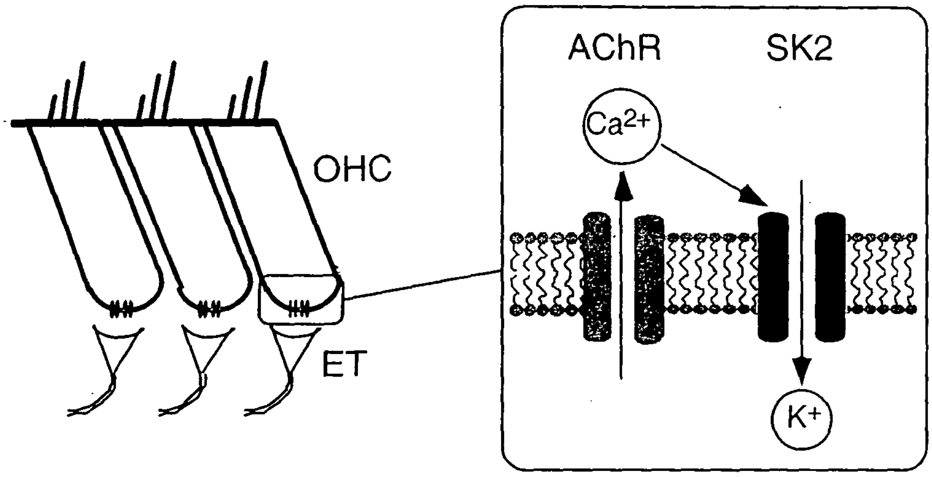
AChR
SK2
Ca2+
OHC
ET
K+

B

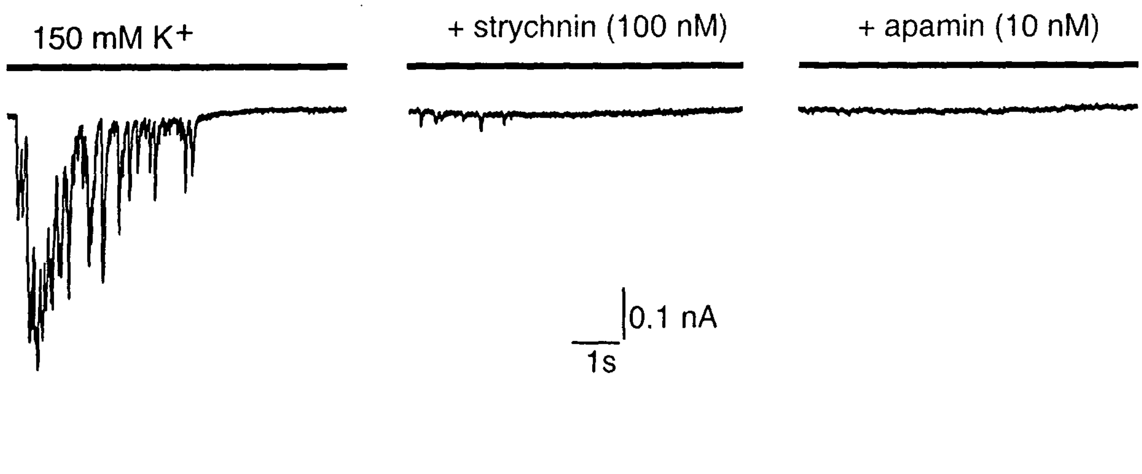
150 mM K+
+ strychnin (100 nM)
+ apamin (10 nM)
0.1 nA
1s

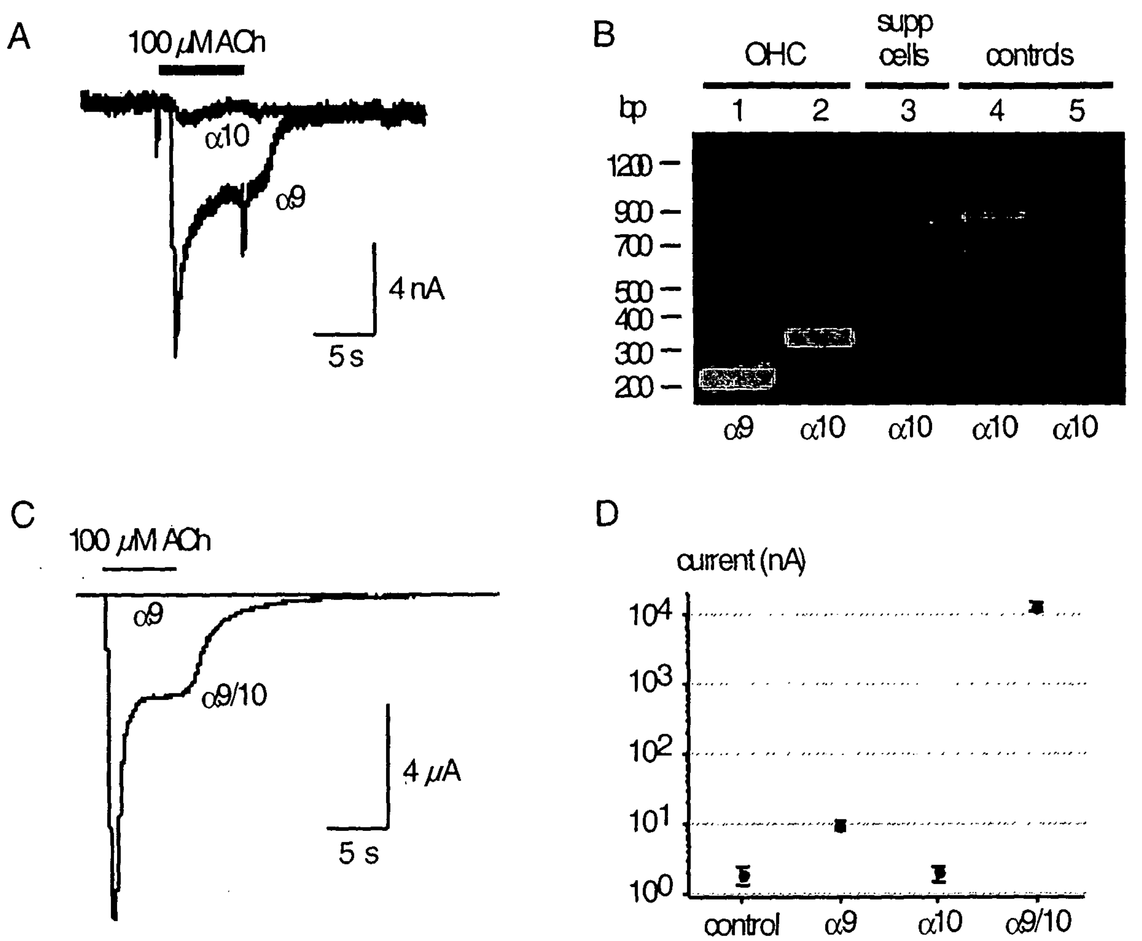
A
100 μM ACh
α10
α9
4 nA
5 s
B
OHC
supp cells
controls
bp
1
2
3
4
5
1200
900
700
500
400
300
200
α9
α10
α10
α10
α10
C
100 μM ACh
α9
α9/10
4 μA
5 s
D
current (nA)
10^4
10^3
10^2
10^1
10^0
control
α9
α10
α9/10

Fig. 2

Fig. 3

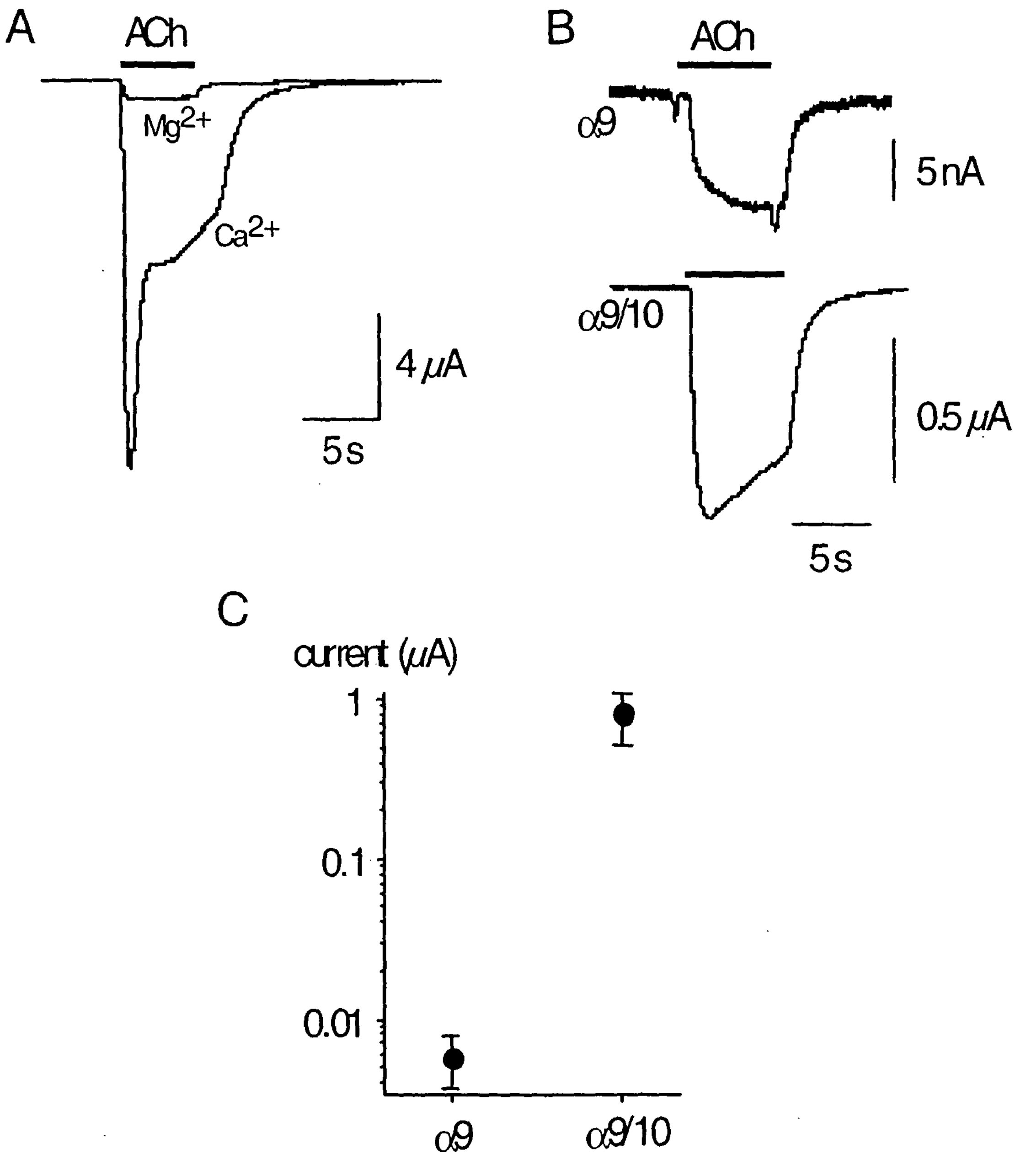
A
ACh
Mg2+
Ca2+
4 μA
5s
B
ACh
α9
5nA
α9/10
0.5 μA
5s
C
current (μA)
1
0.1
0.01
α9
α9/10

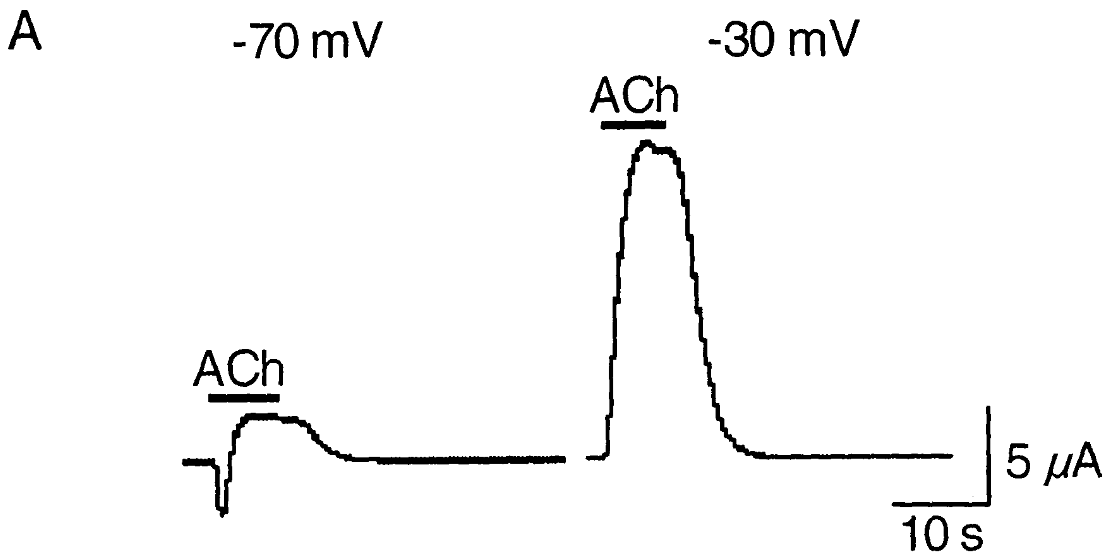


Fig. 4

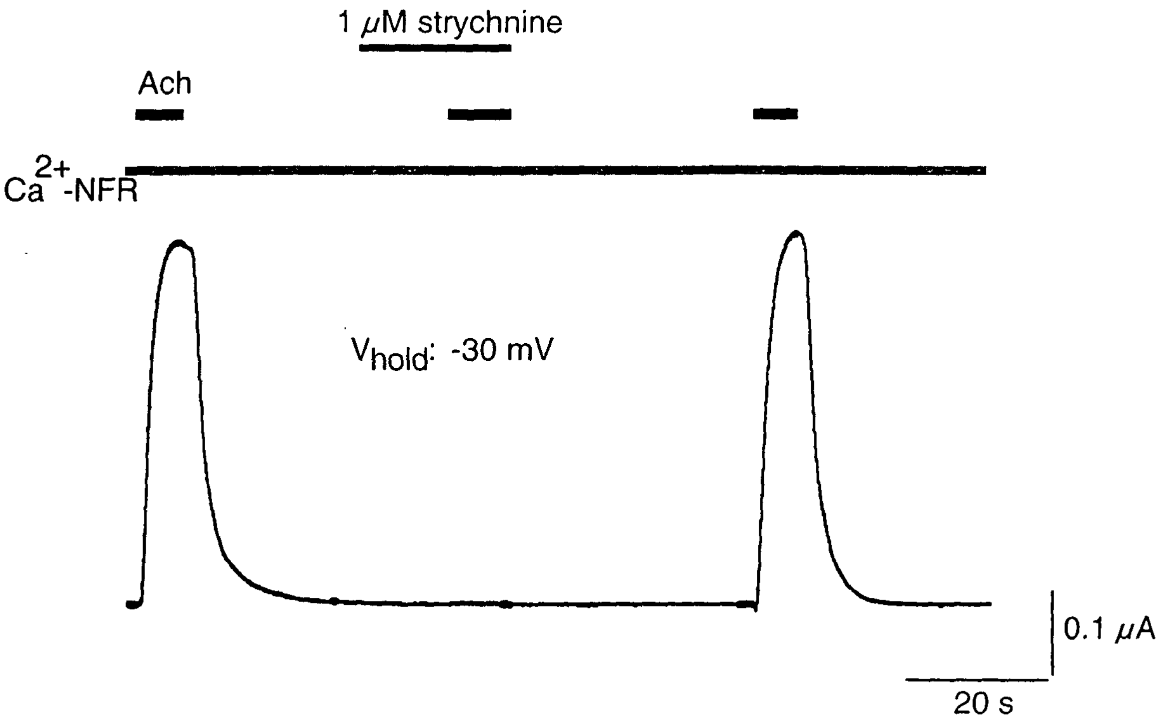


Fig. 5

European Patent Office

# PARTIAL EUROPEAN SEARCH REPORT

which under Rule 45 of the European Patent Convention shall be considered, for the purposes of subsequent proceedings, as the European search report

Application Number EP 05 00 9885

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| P,X | WO 01/23551 A (SANOFI SYNTHELABO ;SGARD FREDERIC (FR); BESNARD FRANCOIS (FR); CHA) 5 April 2001 (2001-04-05)<br>* abstract *<br>* page 26, line 23 - page 28, line 12 *<br>* page 28, line 30 - page 31, line 2; claims 23,26,29 *<br>----- | 1-14,16, 17 | A61K31/00<br>A61K31/475<br>A61K38/10<br>A61P27/16 |
| P,X | WO 00/72835 A (EL KHOURY GEORGE F) 7 December 2000 (2000-12-07)<br>* the whole document, in particular page 3, lines 28-32 *<br>----- | 1,2,4,5, 9-11,13, 17 | |
| X | WO 98/09623 A (SCOTIA HOLDINGS PLC ;BRUNES BIRGITTA (SE); BRUNES CHRISTIAN (SE)) 12 March 1998 (1998-03-12)<br>* the whole document *<br>-----<br>-/-- | 1,2,4,5, 9-11,13, 17 | |

TECHNICAL FIELDS SEARCHED (Int.Cl.7)

A61K
A61P

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

1

| Place of search | Date of completion of the search | Examiner |
|---|---|---|
| The Hague | 7 June 2005 | Hoff, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C07)

European Patent Office

# PARTIAL EUROPEAN SEARCH REPORT

Application Number
EP 05 00 9885

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | WO 96/03504 A (SALK INST FOR BIOLOGICAL STUDI) 8 February 1996 (1996-02-08)<br>* abstract *<br>* page 15, line 16 - line 23 *<br>* page 19, line 24 - page 20, line 29 *<br>----- | 1,2,4,5, 9-11,13, 17 | |
| D,A | ELGOYHEN A B ET AL: "Alpha 9: an acetylcholine receptor with novel pharmacological properties expressed in rat cochlear hair cells."<br>CELL, (1994 NOV 18) 79 (4) 705-15.,<br>18 November 1994 (1994-11-18), XP001002600<br>* the whole document *<br>----- | 1,2,4,5, 9-11,13, 17 | TECHNICAL FIELDS SEARCHED (Int.Cl.7) |
| A | GLOWATZKI E ET AL: "Cholinergic synaptic inhibition of inner hair cells in the neonatal mammalian cochlea."<br>SCIENCE, (2000 JUN 30) 288 (5475) 2366-8.,<br>30 June 2000 (2000-06-30), XP001011319<br>* the whole document *<br>----- | 1-17 | |
| D,A | D. OLIVER ET AL.: "Gating of Ca2+-Activated K+ Channels Controls Fast Inhibitory Synaptic Transmission at Auditory Outer Hair Cells"<br>NEURON,<br>vol. 26, no. 3, June 2000 (2000-06), pages 595-601, XP001005999<br>* the whole document *<br>----- | 1-17 | |
| D,A | EP 0 759 295 A (ZENNER HANS PETER PROF DR MED) 26 February 1997 (1997-02-26)<br>* the whole document *<br>----- | 1-17 | |

1

EPO FORM 1503 03.82 (P04C10)

European Patent Office

## INCOMPLETE SEARCH SHEET C

Application Number
EP 05 00 9885

Reason for the limitation of the search:

Present claims 1-5,10-13 relate to a compound defined by reference to a desirable characteristic or property, namely "substance which is at least partly blocking at least one ionotropic acetylcholine receptor and/or at least one calcium-activated potassium channel functionally associated with an ionotropic acetylcholine receptor of the inner ear".

The claims cover all compounds having this characteristic or property, whereas the application provides support within the meaning of Article 84 EPC and disclosure within the meaning of Article 83 EPC for only a very limited number of such compounds. In the present case, the claims so lack support, and the application so lacks disclosure, that a meaningful search over the whole of the claimed scope is impossible. Independent of the above reasoning, the claims also lack clarity (Article 84 EPC). An attempt is made to define the compound by reference to its pharmacological profile. Again, this lack of clarity in the present case is such as to render a meaningful search over the whole of the claimed scope impossible.

Moreover, present claims 6-9,14,16,17 relate to an extremely large number of possible compounds (any strychnine derivatives, peptides, polynucleotides and small molecular compound having the claimed pharmacological profile). Again, support within the meaning of Article 84 EPC and disclosure within the meaning of Article 83 EPC is to be found, however, for only a very small proportion of the compounds claimed. In the present case, the claims so lack support, and the application so lacks disclosure, that a meaningful search over the whole of the claimed scope is impossible.

Consequently, the search has been carried out for those parts of the claims which appear to be clear, supported and disclosed, namely those parts relating to the compounds mentioned in the examples (strychine and apamine), with due regard to the general idea underlying the present invention.

**ANNEX TO THE EUROPEAN SEARCH REPORT ON EUROPEAN PATENT APPLICATION NO.** EP 05 00 9885

This annex lists the patent family members relating to the patent documents cited in the above-mentioned European search report.
The members are as contained in the European Patent Office EDP file on
The European Patent Office is in no way liable for these particulars which are merely given for the purpose of information.

07-06-2005

| Patent document cited in search report | Publication date | Patent family member(s) | Publication date |
|---|---|---|---|
| WO 0123551 A | 05-04-2001 | EP 1090991 A1 | 11-04-2001 |
| | | AU 6442000 A | 30-04-2001 |
| | | WO 0123551 A1 | 05-04-2001 |
| | | EP 1222265 A1 | 17-07-2002 |
| WO 0072835 A | 07-12-2000 | AU 5170400 A | 18-12-2000 |
| | | WO 0072835 A2 | 07-12-2000 |
| | | US 6262063 B1 | 17-07-2001 |
| | | US 2002010191 A1 | 24-01-2002 |
| WO 9809623 A | 12-03-1998 | AU 4027097 A | 26-03-1998 |
| | | WO 9809623 A2 | 12-03-1998 |
| | | ZA 9707867 A | 27-05-1998 |
| WO 9603504 A | 08-02-1996 | US 5683912 A | 04-11-1997 |
| | | CA 2192694 A1 | 08-02-1996 |
| | | EP 0765391 A1 | 02-04-1997 |
| | | JP 10503091 T | 24-03-1998 |
| | | WO 9603504 A1 | 08-02-1996 |
| | | US 6646109 B1 | 11-11-2003 |
| | | US 6013766 A | 11-01-2000 |
| | | US 6100046 A | 08-08-2000 |
| EP 0759295 A | 26-02-1997 | DE 19528388 A1 | 06-02-1997 |
| | | AT 163545 T | 15-03-1998 |
| | | AU 719018 B2 | 04-05-2000 |
| | | AU 6788296 A | 26-02-1997 |
| | | BR 9609950 A | 29-06-1999 |
| | | CA 2228393 A1 | 13-02-1997 |
| | | CN 1194581 A ,C | 30-09-1998 |
| | | DE 19680619 D2 | 29-10-1998 |
| | | DE 59600105 D1 | 09-04-1998 |
| | | DK 759295 T3 | 25-01-1999 |
| | | WO 9704762 A1 | 13-02-1997 |
| | | EP 0759295 A1 | 26-02-1997 |
| | | EP 0834310 A1 | 08-04-1998 |
| | | ES 2116801 T3 | 16-07-1998 |
| | | IL 123142 A | 31-10-2001 |
| | | JP 3568039 B2 | 22-09-2004 |
| | | JP 2000515486 T | 21-11-2000 |
| | | PL 324795 A1 | 22-06-1998 |
| | | US 6066652 A | 23-05-2000 |

EPO FORM P0459

For more details about this annex : see Official Journal of the European Patent Office, No. 12/82